# FASCICULE DE BREVET EUROPEEN

(11) **EP 1 540 038 B1**
(45) Date de publication et mention de la délivrance du brevet: **11.05.2011**
(21) Numéro de dépôt: 03797338.5
(22) Date de dépôt: 12.09.2003
(51) Int. Cl.: C25B 3/02

(54) **PROCEDE DE PREPARATION D'UNE COMPOSITION POLYCARBOXYLIQUE COMPRENANT UNE ETAPE D'OXYDATION ELECTROCHIMIQUE D'UNE COMPOSITION MONOSACCHARIDIQUE**
VERFAHREN ZUR HERSTELLUNG EINER POLYCARBONSÄUREZUSAMMENSETZUNG MIT EINER STUFE DER ELEKTROCHEMISCHEN OXIDATION EINER MONOSACCHARIDZUSAMMENSETZUNG
METHOD FOR PREPARING A POLYCARBOXYLIC COMPOSITION COMPRISING AN ELECTROCHEMICAL OXIDATION STAGE OF A MONOSACCHARIDE COMPOSITION

(30) Priorité: 18.09.2002 FR 0211546
(43) Date de publication de la demande: 15.06.2005
(73) Titulaire: Roquette Frères, 62136 Lestrem (FR)
(72) Inventeur: MARSAIS, Francis, 76000 Rouen (FR); FEASSON, Christian, 76420 Bihorel Les Rouen (FR); QUEGUINER, Guy, 76420 Bihorel Les Rouen (FR); IBERT, Mathias, 76000 Rouen (FR); COMINI, Serge, 59253 La Gorgue (FR); GROSSEL, Jean-Marc, 59660 Merville (FR)
(74) Mandataire: Boulinguiez, Didier
(86) Numéro de dépôt international: PCT/FR2003/002702
(87) Numéro de publication internationale: WO 2004/027118

(56) Documents cités:
- EP-A- 1 027 931
- WO-A-91/04988
- FR-A- 715 176

## Description

La présente invention a pour objet un nouveau procédé de préparation de compositions polycarboxyliques, ledit procédé comprenant une étape d'oxydation électrochimique d'une composition monosaccharidique, menée dans des conditions particulières.

Elle concerne également, en tant que matières nouvelles, certaines des compositions polycarboxyliques susceptibles d'être obtenues par ledit procédé.

En outre, la présente invention vise plus particulièrement l'utilisation desdites compositions polycarboxyliques dans certaines industries telles que, par exemple, celles des détergents et agents de nettoyage, du traitement des eaux, des liants hydrauliques, les industries alimentaires ou pharmaceutiques.

Dans ces industries, il est fait couramment appel à des matières issues de produits d'origine naturelle ou synthétique, de structure polymérique ou monomérique, et contenant aux moins deux fonctions carboxyliques, tout ou partie desdites fonctions pouvant se présenter sous forme « acide libre » (COOH) ou sous d'autres formes, en particulier de sels associés tels que des sels alcalins ou alcalino-terreux. Ces matières, qualifiables de « polycarboxylates », peuvent notamment être utilisées comme agents chélatants ou séquestrants des métaux, builders ou co-builders de détergence, agents retardateurs de prise de liants hydrauliques mais aussi comme agents stabilisants, structurants, dispersants, désintégrants ou débourrants de compositions de toutes natures et destinations.

Elles peuvent consister en des dérivés de produits naturels tels que les poly- et monosaccharides, en particulier en des dérivés d'amidon ou de produits d'hydrolyse de l'amidon.

Il peut s'agir, entre autres :
- de dérivés carboxyalkylés d'hydrolysats d'amidon,
- de glucuronyl-arabinarates ou de glucuronyl-glucarates, obtenus à partir d'hydrolysats d'amidon,
- d'acide glucuronyl-glucarique, d'acide glucarique ou d'acide mannarique obtenus à partir, respectivement, de maltitol, de sorbitol ou de mannitol,
la préparation et les utilisations de tels polycarboxylates issus de produits naturels étant notamment décrites dans les brevets WO 95/02614, EP 780 399 et EP 798 310 au nom de la Demanderesse ainsi que, par exemple, dans le brevet EP 656 051.

D'autres polycarboxylates, monomériques ou polymériques, sont issus de produits d'origine synthétique ou d'origine naturelle mais de nature non saccharidique tels que, par exemple :
- les acides dicarboxyliques comme les acides tartrique, succinique ou glutarique,
- les acides tricarboxyliques comme les acides citrique ou nitrilotriacétique (« NTA »),
- les acides tétracarboxyliques comme l'acide éthylènediamine tétra-acétique (« EDTA »),
- les (co)-polymères des acides carboxyliques éthyléniques tels que, par exemple, les polyacrylates,
les utilisations de tels polycarboxylates issus de produits de nature non saccharidique étant, entre autres, décrites dans les brevets FR 2 657 601, FR 2 735 788, WO 91/00901, EP 565 266, EP 605 318, EP 650 941 ou EP 972 825.

Compte-tenu des contraintes actuelles en termes de protection de l'homme et de son environnement, il est souhaité de pouvoir disposer de polycarboxylates issus de produits d'origine naturelle et renouvelables.

Parmi de tels polycarboxylates, l'acide glucarique et ses sels ont fait l'objet de nombreux travaux depuis une décennie environ.

Le brevet EP 656 051 précité décrit la préparation de détergents sans phosphates à base de zéolithes et/ou de silicates lamellaires contenant, en tant qu'agents complexants, des acides ou sels d'acides polyhydroxydicarboxyliques comportant de 4 à 6 atomes de carbone et au moins 2 groupements hydroxyles par molécule tels que notamment les glucarate et galactarate de sodium.

Le brevet EP 798 310 précité, publié au nom de la Demanderesse, décrit, dans son Exemple 1, la préparation d'une composition contenant 33 % d'acide glucarique sous forme de son sel de sodium et 67 % de chlorure de sodium, avec absence de produits de sur-oxydation de l'acide glucarique. Cette composition, très riche en NaCl, est obtenue par oxydation de sorbitol par l'hypochlorite de sodium (NaOCl ou « eau de javel ») en présence d'un catalyseur constitué par un nitroxy alkyle binaire ou tertiaire tel que le 2,2,6,6-tétraméthylpipéridinyloxy ou « TEMPO ».

D'autres travaux ont été décrits plus récemment dans l'article de J. F. THABURET et al. intitulé « TEMPO-mediated oxidation of maltodextrins and D-glucose : effect of pH on the selectivity and sequestering ability of the resulting polycarboxylates », Carbohydrate Research, 330 (2001), pp. 21-29.

Ces travaux ont montré que l'oxydation du glucose ou du sorbitol en présence de TEMPO et de NaOCl était particulièrement difficile à contrôler mais qu'il était possible cependant, sous certaines conditions très précises (dont pH de l'ordre de 11,7 et nécessité de présence de bromure de sodium NaBr), d'obtenir un bon rendement (90 %) en acide glucarique.

Ce document montre par ailleurs qu'en fonction du nombre d'équivalents de NaOCl mis en oeuvre, cette synthèse d'acide glucarique est accompagnée de la co-production préférentielle :
- soit d'acide gluconique, i.e. de l'acide monocarboxylique correspondant à l'acide glucarique,
- soit d'autres acides dicarboxyliques mais de petite taille, à savoir l'acide tartrique et l'acide oxalique.

Les auteurs attribuent la coproduction de ces deux derniers acides par la dégradation oxydative du monosaccharide (glucose, sorbitol) en deux endroits de la molécule, à savoir a) entre les atomes de carbone C-4 et C-5 et b) entre les atomes de carbone C-2 et C-3.

Encore plus récemment, d'autres voies de dégradation de l'acide glucarique ont été proposées, notamment pour tenter d'expliquer la co-production d'acide méso-tartrique ou d'acide D-tartrique lors de l'oxydation du glucose en acide glucarique en présence de NaOCl, de NaBr et de TEMPO.

Ces voies de dégradation, hypothétiques ou non, sont tout particulièrement décrites dans l'article très récent de M. IBERT et al. intitulé « Détermination of the side-products formed during the nitroxide-mediated bleach oxidation of glucose to glucaric acid », Carbohydrate Research, 337 (2002), pp. 1059-1063.

En tout état de cause, il résulte de ce qui précède que la préparation d'acide dicarboxylique comme l'acide glucarique à partir d'un monosaccharide comme le glucose, éventuellement hydrogéné (sorbitol), engendre un certain nombre d'inconvénients et notamment :
- la mise en oeuvre d'hypochlorite de sodium, non souhaitable compte-tenu des contraintes actuelles en termes de protection de l'homme et de son environnement,
- la mise en oeuvre éventuelle de NaBr, non souhaitable car également susceptible de générer des espèces halogénées,
- l'obligation de bien contrôler et maîtriser des paramètres réactionnels tels que le pH et la concentration en NaOCl aux fins d'obtenir un rendement minimum ou acceptable en produit souhaité,
- la co-production significative et non souhaitée soit de matières non économiquement rentables (NaCl) soit de matières de nature monocarboxylique (acide gluconique et ses sels) soit de matières de nature dicarboxylique (acides oxalique et tartrique et leurs sels) mais dont les molécules présentent un poids moléculaire (très) fortement réduit dû à une dégradation et portent peu (acide tartrique) ou pas (acide oxalique) de groupements OH et dont la présence diminue donc les propriétés, en particulier chélatantes ou séquestrantes, de la composition d'acide glucarique.

Outre ces problèmes de genèse de produits de dégradation, l'oxydation de monosaccharides en présence de TEMPO et d'hypochlorite de sodium, ne permet pas d'obtenir des matières présentant trois fonctions carboxyliques comme c'est le cas, par exemple, de polycarboxylates couramment utilisés dans l'industrie comme l'acide citrique et les citrates.

Il existait donc un besoin de disposer d'un moyen apte à s'affranchir des inconvénients précités et notamment d'un moyen qui, à partir de monosaccharides, permette a) de préparer efficacement des compositions présentant une richesse élevée en matières polycarboxyliques, et notamment une richesse élevée en matières dicarboxyliques comme l'acide glucarique et ses sels et b) éventuellement, de disposer de nouvelles compositions à base non seulement de matières dicarboxyliques mais également de matières tricarboxyliques, l'ensemble de ces compositions pouvant être avantageusement utilisées dans les industries et pour les fonctionnalités préalablement mentionnées.

Ceci, sans avoir besoin de mettre en oeuvre d'hypochlorite de sodium et de contrôler très rigoureusement le pH réactionnel.

Et le mérite de la Société Demanderesse est d'avoir trouvé, après de nombreux travaux de recherche, qu'un tel moyen pouvait consister en la mise en oeuvre d'un procédé particulier, en l'occurrence un procédé d'oxydation électrochimique, mené dans des conditions particulières, notamment liées à la nature spécifique de l'anode utilisée en vue de ladite oxydation.

Plus précisément la présente invention a pour objet un procédé de préparation d'une composition polycarboxylique caractérisé par le fait qu'il comprend une étape au cours de laquelle on soumet une composition monosaccharidique à un traitement d'oxydation électrochimique mené en absence d'hypochlorite de sodium et en présence a) d'un oxyde d'amine et b) d'une anode à base de matériau en carbone.

Par « composition polycarboxylique » au sens de la présente invention, on entend ici toute composition contenant au moins 50 % en poids, d'un ou plusieurs produits choisis dans le groupe comprenant les acides dicarboxyliques, les acides tricarboxyliques, les sels et les dérivés desdits acides, ce pourcentage étant exprimé en poids sec total de ce(s) produit(s) par rapport au poids sec total de ladite composition.

Par « composition monosaccharidique » on entend ici toute composition contenant au moins 50 % en poids, d'un ou plusieurs monosaccharide(s), ce pourcentage étant exprimé en poids sec total de monosaccharide(s) par rapport au poids sec total de ladite composition.

Ce pourcentage peut avantageusement être d'au moins 80 % et atteindre 100 %.

La matière sèche de ladite composition monosaccharidique peut se situer dans des gammes très larges et notamment entre 0,1 et 50 %, en fonction notamment d'éventuelles contraintes d'ordre économique ou technique, par exemple liées à l'optimisation de la viscosité et des caractéristiques thermiques du milieu réactionnel.

Cette matière sèche (MS) peut notamment être constituée par au moins un monosaccharide choisi dans le groupe comprenant les aldoses, les cétoses et leurs dérivés respectifs à l'exception de ceux dont la fonction hémiacétalique portée par le carbone en position 1 (C1) a été protégée contre l'oxydation par amination, estérification, étherification, acétalisation ou greffage. De tels dérivés ainsi « protégés » en C1, non utilisables en tant que « monosaccharides » dans le cadre de la présente invention, consistent par exemple en méthyl-α-glucopyranoside, isopropyl(α,β)-D-glucopyranoside , α-D-glucose pentaacétate, (α,β)-D-glucopyranosyl phosphate. L'oxydation par voie électrochimique de tels dérivés génère des acides uroniques, i.e. des matières monocarboxyliques dont seule la fonction alcoolique primaire portée par le carbone en position 6 (C6) du glucose a été oxydée.

L'obtention de tels acides uroniques par oxydation électrochimique de monosaccharides est largement décrite dans la littérature et en particulier dans le brevet EP 1027 931 et les articles suivants :
- « Catalytic oxidation of sugars by 4-(acetylamino)-TEMPO », K. ITO et al., Proc. Electrochem. Soc., 1993, Vol. 93-11, pp. 260-267,
- « Electroorganic Synthesis 66 : Selective Anodic Oxidation of Carbohydrates Mediated by TEMPO », K. SCHNATBAUM et al., Synthesis, 1999, N° 5, pp. 864-872,

Dans le cadre de la présente invention, tout constituant de la composition monosaccharidique soumise à une étape d'oxydation électrochimique peut notamment être choisi dans le groupe comprenant :
- les aldohexoses tels que le glucose, le mannose, le galactose ou le gulose,
- les aldopentoses et aldotétroses tels que le ribose, l'arabinose, le xylose, l'érythrose et le thréose,
- les cétoses tels que le fructose, le tagatose, le sorbose et le xylulose
- les monosaccharides résultant de l'hydrogénation des produits précités tels que le sorbitol, le mannitol, le galactitol, le xylitol, le ribitol, l'arabitol, l'érythritol et le thréitol,
- les monosaccharides résultant de l'oxydation des produits précités tels que l'acide gluconique, les acides cétogluconiques, l'acide glucarique, l'acide galactonique, l'acide xylonique ou l'acide arabinonique, leurs lactones et sels correspondants,
- les autres dérivés des produits précités dès lors qu'ils ne sont pas « protégés » en C1 de la façon indiquée précédemment. Il peut s'agir, à titre d'exemple, du 3-méthoxyglucose.

La composition monosaccharidique utilisée comme matière première dans le cadre de l'invention peut, bien que cela ne soit pas préféré, contenir en faibles pourcentages des produits autres que des monosaccharides tels que des di-, oligo- et polysaccharides ou leurs dérivés.

Comme indiqué, de manière préférentielle, la composition monosaccharidique contient au moins 80 % en poids (sec / sec) de monosaccharides(s). Il peut s'agir, à titre purement d'exemples, de compositions contenant 100 % en poids (sec / sec) soit de glucose, soit d'un mélange glucose / acide gluconique ou l'un de ses sels, soit d'acide 5-cétogluconique, soit de sorbitol ou de thréitol.

Les sels de monosaccharides oxydés tels que par exemple les gluconates, cétogluconates ou glucarates de sodium ou potassium, présentent l'intérêt d'augmenter la conductivité du milieu réactionnel et, dans cette fonctionnalité, de se substituer au bromure de sodium.

Par « oxyde d'amine » au sens de la présente invention, on entend en particulier tous les composés décrits dans l'un ou l'autre des brevets EP 780 399, EP 798 310 ou EP 1 027 931 précités et utilisables comme catalyseurs d'oxydation.

Le traitement d'oxydation électrochimique auquel est soumis la composition monosaccharidique peut être mené de toute manière accessible à l'homme de l'art et notamment selon les modalités générales ou certaines variantes exposées dans le brevet EP 1027 931 et/ou l'article de K. ITO précités, en ce qui concerne :
- la nature du catalyseur (oxyde d'amine) mis en oeuvre, lequel peut notamment consister en TEMPO ou dérivés de celui-ci, éventuellement absorbé en tout ou partie sur un support,
- la température réactionnelle, laquelle peut notamment être inférieure à 30°C, notamment comprise entre 1 et 20°C,
- la nature de la cathode du dispositif d'électrooxydation, laquelle peut être notamment à base de platine, à base de titane, à base d'acier inoxydable ou à base d'un matériau en carbone.

Selon une caractéristique essentielle de l'invention, l'anode en présence de laquelle la composition monosaccharidique est oxydée est cependant spécifiquement à base d'un matériau en carbone.

Par « matériau en carbone », on entend notamment le carbone cristallisé tel que le graphite ou le carbone amorphe tel que le charbon et ses formes activées. Ce matériau peut notamment être utilisé sous forme de barre(s), de billes, de plaques, de grilles, de feutres ou de tampons.

Le catalyseur (oxyde d'amine) peut d'ailleurs, en tout ou partie, être immobilisé, adsorbé ou absorbé au niveau même de ce matériau dès avant l'étape proprement dite d'oxydation.

De manière avantageuse et notamment par souci de productivité, ce matériau présente une surface spécifique élevée, i.e. au moins égale à 0,10 m²/g, de préférence au moins égale à 0,20 m²/g. Il peut s'agir, à titre d'exemples, d'un feutre de carbone ou de charbon actif granulaire tel que celui de type « NORIT AX 08 ».

De façon particulièrement avantageuse, ledit matériau peut présenter une surface spécifique au moins égale à 0,25 m²/g.

La Société Demanderesse a constaté que, de façon surprenante et inattendue, l'utilisation d'une anode à base de matériau en carbone dans un système exempt d'hypochlorite de sodium, permettait d'obtenir des rendements élevés, i.e. supérieurs à 50 % et y compris supérieurs à 90 %, en matières dicarboxyliques telles que l'acide glucarique et ses sels.

Ce résultat est d'autant plus remarquable que de tels rendements peuvent être obtenus dans une gamme de pH réactionnels relativement large, i.e. entre 11 et 14, y compris donc pour des valeurs de pH égales ou supérieures à 12, valeurs jamais véritablement exemplifiées dans l'art antérieur précité.

Selon une variante du procédé selon l'invention le traitement d'oxydation électrochimique est mené à un pH compris entre 10 et 14, de préférence entre 11,5 et 14.

Par ailleurs et de manière encore plus surprenante, le procédé selon l'invention permet d'obtenir directement, i.e. sans étape de purification, des compositions polycarboxyliques contenant des taux significatifs non seulement en matières dicarboxyliques (par exemple, en acide glucarique et/ou ses sels) mais également en matières tricarboxyliques. Ces taux en matières tricarboxyliques peuvent notamment se situer entre 3 et 50 % en poids (sec / sec).

Selon l'invention, la composition polycarboxylique obtenue à l'issue de l'étape d'oxydation électrochimique de la composition monosaccharidique, contient :
- de 30 à 90 % d'un ou plusieurs produits choisis parmi les acides dicarboxyliques et leurs sels, et
- de 3 à 50 % d'un ou plusieurs produits choisis
   parmi les acides tricarboxyliques et leurs sels,
   ces pourcentages étant exprimés en poids secs par rapport au poids sec total de ladite composition.

A la connaissance de la Demanderesse, il n'a jamais été obtenu, a fortiori décrit, l'obtention de telles compositions à partir de compositions monosaccharidiques, y compris de telles compositions polycarboxyliques dont le composant dicarboxylique est composé d'acide glucarique et/ou de ses sels.

De telles compositions consistent, en de nouveaux produits industriels qui peuvent, être avantageusement utilisées en particulier dans les industries des détergents et agents de nettoyage, du traitement des eaux, du traitement des métaux, du traitement des végétaux, du traitement des fibres, en particulier textiles ou papetières, des liants hydrauliques, des adhésifs, de la fonderie, des peintures ou du cuir. Ces compositions peuvent également être utilisées dans les industries alimentaires, pharmaceutiques ou cosmétologiques.

Il a notamment été observé que le procédé selon l'invention permettait d'obtenir, à partir d'hexoses, des compositions polycarboxyliques contenant, sous forme d'acide libre et/ou de sel(s), un composé de structure nouvelle, présentant trois fonctions carboxyliques et six atomes de carbone.

Après de longs travaux de recherche et d'analyse, la Demanderesse propose de retenir la dénomination de « acide 2-carboxy-2,3,4-trihydroxypentanedioïque » pour ledit composé, lequel répondrait à la formule plane générale suivante : 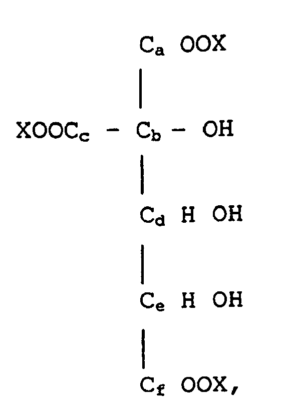 pour laquelle :
- chacun des 3 éléments « X » peut notamment être choisi dans le groupe comprenant l'hydrogène, les métaux et en particulier les métaux alcalins et alcalino-terreux, les groupements aminés et en particulier le groupement ammonium, les groupements alcoyle et en particulier éthyle et méthyle et les groupements silylés, et
- chacun des groupements OH porté soit par l'atome de carbone C_{d} soit par l'atome de carbone Ce, peut se situer soit à droite soit à gauche du squelette carboné.

En fonction de la localisation possible de chacun de ces deux groupements OH particuliers, ce composé doit être considéré ici comme divulgué pour chacun de ses quatre isomères (1), (2), (3) et (4) décrits ci-dessous (pour lesquels chaque élément « X » est tel que défini précédemment) mais également pour tout mélange quelconque d'au moins deux quelconques de ces isomères.

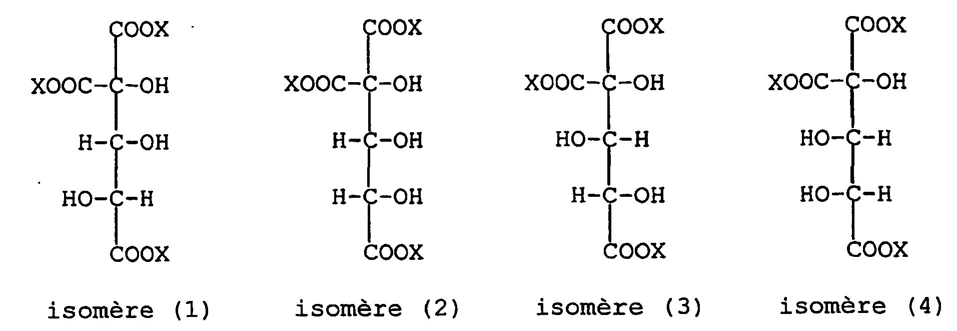

Comme il sera exemplifié par ailleurs, le procédé conforme à l'invention permet, en outre, en fonction de la nature de la composition monosaccharidique mise en oeuvre, d'obtenir des compositions polycarboxyliques contenant :
- soit l'un seulement des quatre isomères sus-décrits, par exemple, uniquement l'isomère(1) quand la composition monosaccharidique est une composition de D-galactose ou d'acide D-5-cétogluconique ou uniquement l'isomère (2) quand il s'agit d'une composition de D-fructose, de D-mannose ou d'acide D-2-cétogluconique,
- soit un mélange en toutes proportions d'au moins deux des quatre isomères sus-décrits, par exemple un mélange contenant majoritairement l'isomère (1) et minoritairement l'isomère (2) quand il s'agit d'une composition de D-glucose.

A la connaissance de la demanderesse, aucun desdits isomères n'a jamais été synthétisé, a fortiori identifié, y compris dans l'art antérieur précité. A ce titre, il convient d'insister sur le fait qu'aucun de ces isomères n'a jamais été répertorié par Chemical Abstract Service (CAS) et ne peut donc avoir reçu un numéro d'enregistrement « CAS ».

Il apparaît que, parmi les très nombreuses possibilités d'oxydation d'hexoses par voie chimique ou électrochimique, seul le procédé ici revendiqué combinant 1) l'absence de NaOCl et 2) une oxydation électrochimique en présence d'une anode à base de matériau en carbone, peut permettre d'obtenir l'un au moins des isomères de l'acide 2-carboxy-2,3,4-trihydroxypentanedioïque, sous forme libre, de sel(s) et/ou autres.

La demanderesse n'a notamment pas observé de formation dudit acide ou de ses sels en substituant ladite anode par une anode à base d'autres matériaux, en particulier à base d'acier inoxydable.

Il est remarquable de souligner que le procédé selon l'invention permet d'obtenir, avec de bons rendements, des compositions polycarboxyliques riches en produits à la fois « suroxydés » (di ou tricarboxylés) et non dégradés, i.e. contenant le même nombre d'atomes de carbone que le monosaccharide de départ.

La Demanderesse a notamment observé que le traitement d'oxydation électrochimique conforme à l'invention, appliqué à des hexoses et à un pH situé entre 12 et 13,5 environ permettait d'obtenir directement des compositions polycarboxyliques conformes à l'invention telles que décrites ci-avant et dont la teneur totale en acide glucarique (sous forme acide libre et/ou ses sels) et acide 2-carboxy-2,3,4-trihydroxypentanedioïque (sous forme acide libre et/ou de sels) était au moins égale à 90 %, ce pourcentage étant exprimé en poids sec total desdits produits par rapport aux poids sec total de composition.

Sans vouloir être liée par une quelconque théorie, la Demanderesse pense que dans les conditions sélectionnées conformément au procédé selon l'invention (absence de NaOCl, anode à base de matériau en carbone notamment) et en particulier lorsque ledit procédé est mené à un pH situé dans la gamme 12 - 13,5 environ, une partie de l'acide gluconique, éventuellement utilisé comme matière première mais plus généralement obtenu comme produit intermédiaire à partir de glucose, se transforme significativement et rapidement en acide glucarique avant d'évoluer significativement mais progressivement (par exemple en 15 heures) en acide 2-carboxy-2,3,4-trihydroxypentanedioïque et ce, par réarrangements successifs faisant vraisemblablement intervenir en particulier les acides 5-cétogluconique et 2-cétogluconique comme certains des produits intermédiaires et générant peu ou pas de produits de dégradation comportant de 2 à 5 atomes de carbone.

Et il est remarquable de noter, comme l'a constaté la Demanderesse, que les effets ou résultats générés par le procédé conforme à l'invention et décrits ci-avant peuvent être obtenus tant en présence qu'en absence de bromure de sodium, précurseur du co-oxydant dont l'usage est largement préconisé dans l'art antérieur lors du traitement d'oxydation (électro)chimique mais qui génère, comme NaOCl, des espèces halogénées indésirables.

La présente invention va être décrite de façon encore plus détaillée à l'aide des exemples qui suivent et qui ne sont aucunement limitatifs.

### EXEMPLE 1

Dans un réacteur d'électrolyse connecté à un système de contrôle de pH et de température et équipé d'une anode constituée d'un feutre de carbone d'une épaisseur de 5 mm et d'une surface spécifique de 0,3 m²/g fourni par la Société CARBONE-CORRBINE et d'une cathode à base d'acier inoxydable, on introduit 170 ml d'eau osmosée (2 Mohm.cm), 20 g (0,099 mole) de D-glucose sous forme de dextrose monohydrate et 80 mg (0,51 mole) de « TEMPO » (2,2,6,6-tétraméthylpipéridinyloxy) .

L'électrolyse est réalisée à intensité constante (600 mA), en maintenant la température du milieu réactionnel entre 2 et 5°C, et en régulant le pH à 12,2 à l'aide d'une solution de potasse 4M. La tension d'électrolyse (entre anode et cathode) varie de 5V en début d'électrolyse à 3V en fin de réaction. L'électrolyse est arrêtée lorsque la quantité d'électricité délivrée correspond à 125 % de la quantité théorique nécessaire à l'oxydation du glucose en acide glucarique (6 moles d'électrons par mole de glucose).

La composition obtenue directement à l'issue de cette étape d'oxydation électrochimique (ci-après « COMPOSITION 1 ») est concentrée à 100 ml sous vide, puis amenée à pH 3,8 par addition progressive de résine Amberlite IR-120, et le milieu est agité pendant 1 heure à température ambiante.

La matière précipitée et la résine sont filtrée sur du verre fritté de porosité 4.

Le filtrat résultant est amené à pH 9 par addition de potasse 4 M, évaporé à sec sous vide**(*)** puis séché sous vide**(*)** à 50°C. On obtient ainsi 9,9 g d'une matière solide blanche (ci-après « COMPOSITION 1A »).

Le mélange de matière précipitée et de résine est repris par 60 ml d'eau osmosée (2 Mohm.cm)et son pH est porté à 9 par ajout de potasse 4M. La résine est ensuite éliminée par filtration sous vide.

Le filtrat résultant est évaporé à sec sous vide **(*)** puis séché sous vide **(*)** à 50°C.

### (*) sous pression de 14 mm de mercure (14 mm Hg)

On obtient ainsi 21,5 g d'une matière solide blanche (ci-après « COMPOSITION 1B »).

Les COMPOSITIONS 1, 1A et 1B ont été analysées par chromatographie en phase gazeuse sur colonne capillaire après persilylation. Les espèces sont repérées par le temps de rétention et quantifiées en utilisant un étalon interne et des composés de référence.

La structure et l'identité de certaines espèces ont été recherchées au moyen de :
- la spectrométrie de masse couplée à la chromatographie en phase gazeuse en utilisant plusieurs types de dérivés et en opérant en mode impact électronique ou en mode ionisation chimique,
- la spectrométrie de Résonance Magnétique Nucléaire du proton et du carbone 13.

Les techniques d'analyse utilisées étaient celles décrites dans l'article de M. IBERT précité.

Après de nombreux travaux de recherche et d'analyses qualitatives et quantitatives, la Demanderesse a trouvé que la COMPOSITION 1B contenait en poids (sec / sec), environ :
- 93,7 % de glucarate de potassium,
- 0,3 % de gluconate de potassium,
- 0,3 % de tartrate de potassium,
- 0,3 % de tartronate de potassium,
- 0,5 % d'oxalate de potassium, et
- 5,0 % d'un sel de potassium d'un acide tricarboxylique jusqu'alors inconnu (ci-après « LE PRODUIT X »).

Pour sa part, la COMPOSITION 1A contenait en poids (sec / sec), environ :
- 65,2 % dudit PRODUIT X, sous sa forme de sel tripotassique,
- 18,2 % de glucarate de potassium,
- 2,5 % de gluconate de potassium,
- 2,4 % de tartrate de potassium,
- 3,5 % de tartronate de potassium, et
- 8,2 % d'oxalate de potassium.

Pour sa part, la COMPOSITION 1 obtenue directement à l'issue de l'étape d'oxydation électrochimique et donc aucunement purifiée, contenait notamment environ 70 % en poids (sec / sec) de glucarate de potassium et 24 % en poids (sec / sec) dudit PRODUIT X sous sa forme de sel tripotassique.

Après de nombreux autres travaux de recherche, la Demanderesse a réussi à purifier le PRODUIT X contenu dans la COMPOSITION 1A.

Ladite composition, amenée à une matière sèche de 29 %, a été traitée sur une colonne de résine de type « PCR 532 » sous forme H+ puis amenée à pH 9 à l'aide d'une solution de potasse 2M et précipitée par mise en oeuvre d'une solution saturée de chlorure de calcium. Après filtration, le précipitat a été amené de nouveau sous sa forme acide par addition de résine de type « CA 200 » jusqu'à redissolution totale.

La résine a ensuite été éliminée par filtration et le filtrat, ramené à pH 9 à l'aide d'une solution de potasse 2M, a été évaporé à sec.

Le produit X (« acide 2-carboxy-2,3,4-trihydroxypentanedioïque ») a ainsi été totalement purifié, en l'occurrence sous forme de tricarboxylate de potassium.

De nombreux travaux d'analyses complémentaires ont permis alors de pouvoir constater que ledit PRODUIT X était en fait constitué d'un mélange contenant majoritairement (> 80 % en poids, en sec / sec) l'isomère (1) sus-décrit et minoritairement (< 20 % en poids, sec / sec) l'isomère (2)sus-décrit.

Par ailleurs, des essais d'électrooxydation de D-glucose en présence de TEMPO ont été menés dans les mêmes conditions que celles décrites ci-avant si ce n'est que l'on a substitué l'anode à base de feutre de carbone par une anode constituée soit de barres en acier inoxydable soit d'un tampon en acier inoxydable.

De manière surprenante et inattendue, il a été observé, après 24 heures d'électrolyse dans ces conditions, que :
1) l'anode constituée de barres en acier inoxydable ne permettait pas de conversion substantielle du glucose, et
2) l'anode constituée d'un tampon en acier inoxydable ne permettait qu'une faible conversion du glucose, cette conversion générant essentiellement des produits de dégradation tels que les acides oxalique et tartrique.

En tout état de cause, dans les deux cas, la Société Demanderesse n'a pas observé la formation d'un quelconque isomère de l'acide 2-carboxy-2,3,4-trihydroxypentanedioique ou d'un quelconque sel d'un tel produit.

### EXEMPLE 2

La Société Demanderesse a par ailleurs testé l'efficacité de la COMPOSITION 1A en tant que co-builder de détergence dans une formulation de poudre compacte contenant 25 % en poids de zéolithes.

Il a été trouvé que ladite COMPOSITION 1A pouvait notamment être utilisée ici efficacement pour son aptitude à réduire la précipitation des sels de calcium et de magnésium et ce, tant à 20°C qu'à 40°C ou 60°C.

Dans cette fonctionnalité, la COMPOSITION 1A s'est révélée globalement plus efficace, par exemple, que des agents complexants synthétiques du commerce tels que les sels de sodium des acide iminodisuccinique (« IDS ») ou éthylène diamine disuccinique (« EDDS »).

En outre, il a été observé que la COMPOSITION 1A pouvait, au sein de la même poudre détergente compacte, être avantageusement utilisée en association avec d'autres co-builders tels que les phosphonates ou tripolyphosphates de sodium, ces associations révélant notamment des effets de synergie en termes de réduction de la précipitation des sels de calcium et magnésium.

Par ailleurs, la COMPOSITION 1A s'est révélée utile comme agent stabilisant du peroxyde d'hydrogène mis en oeuvre dans le traitement des pâtes papetières. Ceci, en particulier par son action complexante du cuivre, ce métal étant alors moins apte à dégrader le peroxyde d'hydrogène.

### EXEMPLE 3

Dans le cadre de ces essais, on a opéré selon le descriptif général de l'EXEMPLE 1 si ce n'est que, dans le cas présent a) on a travaillé avec un excès faradique de 20 % sous 600 mA et un temps d'électrolyse de 6h30 et b) on a fait varier le pH réactionnel entre 11 et 13,5 par incrément de 0,5 unité de pH.

Pour chaque pH réactionnel étudié, on a mesuré les taux, exprimés en % (sec / sec) et en produit sous forme acide, des 4 produits suivants :
- acide gluconique (ci-après « ACIDE A »),
- acide glucarique (ci-après « ACIDE B »),
- acide 2-carboxy-2,3,4-trihydroxypentanedioïque (ci-après « ACIDE C »),
- acide oxalique (ci-après « ACIDE D »).

On a obtenu les résultats ci-dessous, les chiffres après la virgule étant arrondis au pour cent supérieur pour ceux au moins égaux à 0,50 et au pour cent inférieur pour ceux inférieurs à 0,50.

| pH Taux (%) | 11 | 11,5 | 12 | 12,5 | 13 | 13,5 |
|---|---|---|---|---|---|---|
| ACIDE A | 13 | 6 | 3 | 2 | 1 | 0 |
| ACIDE B | 57 | 68 | 77 | 76 | 78 | 82 |
| ACIDE C | 23 | 21 | 17 | 18 | 16 | 10 |
| ACIDE D | 4 | 2 | 1 | 2 | 3 | 2 |

Une autre série d'essais a été réalisée dans les mêmes conditions que celles décrites ci-dessus si ce n'est qu'en outre on a introduit dans le milieu réactionnel de départ du bromure de sodium (NaBr) et ce, en une quantité correspondant à 50 % (sec / sec) de la quantité de glucose mise en oeuvre.

On a obtenu globalement, sur la gamme de pH étudiée, le même niveau et la même évolution du taux respectif de chacun des 4 acides que ceux observés en absence de NaBr.

En outre, un essai mené à un pH de 10,5 en présence de NaBr a permis d'obtenir une composition polycarboxylique contenant notamment environ (sec / sec) 25 % d'acide gluconique (ACIDE A), 37 % d'acide glucarique (ACIDE B), 22 % d'acide 2-carboxy-2,3,4-trihydroxypentanedioïque (ACIDE C) et 7 % d'acide oxalique (ACIDE D), le reste étant essentiellement constitué d'acides tartronique et tartrique.

Ces résultats montrent globalement que le procédé conforme à l'invention permet d'obtenir, à partir de compositions monosaccharidiques, des compositions polycarboxyliques riches en produits à la fois suroxydés (di ou tricarboxylés) et non dégradés, en particulier des compositions :
- dont la teneur totale (sec / sec) en acide glucarique est d'au moins 50 %, de préférence d'au moins 70 %, et/ou
- dont la teneur (sec / sec) en acide glucarique et en acide 2-carboxy-2,3,4-trihydroxypentanedioïque est d'au moins 80 %, de préférence d'au moins 90 %.

Il est surprenant de constater que de tels rendements en produits suroxydés / non dégradés peuvent être obtenus dans une gamme de pH réactionnels relativement large, i.e entre 11 et 14.

En outre, il est remarquable de souligner que pour les valeurs de pH égales ou supérieures à 12, situées en particulier entre pH 12 et 13,5, on peut obtenir simultanément a) une teneur en acide glucarique dépassant 75 % (sec / sec), par exemple de 77 à 82 %, et b) une teneur totale en acide glucarique et acide 2-carboxy-2,3,4-trihydroxypentanedioïque dépassant 90 % (sec / sec), par exemple de 92 à 94 %.

Les mêmes constatations générales ont été faites en substituant le « TEMPO » par des dérivés de celui-ci tels que :
- le 4-Acétylamino-2,2,6,6-tétraméthylpipéridinyloxy,
- le 4-Méthoxy-2,2,6,6-tétraméthylpipéridinyloxy,
- le 2-Hydroxyméthyl-7,7,9,9-tétraméthyl-1,4-dioxa-8-aza-spiro(4.5)decan-8-oxy,
- le 2-Méthoxyméthyl-7,7,9,9-tétraméthyl-1,4-dioxa-8-aza-spiro(4.5)decan-8-oxy,
- le 7,7,9,9-Tétraméthyl-1,4-dioxa-8-aza-spiro(4.5)decan- . 8-oxy.

Les mêmes constatations générales ont été faites en utilisant un dispositif de type « PRIAM 1-2C » tel que commercialisé par la Société SOCEM-ELEC comme réacteur électrochimique.

En outre, des essais complémentaires visant à substituer le D-glucose (hexose) par un pentose, en l'occurrence le D-xylose, le D-arabinose ou le D-ribose, ou par un tétrose, en l'occurrence un tétrose hydrogéné comme le D-L thréitol, ont confirmé l'obtention de compositions polycarboxyliques contenant :
- majoritairement, le produit dicarboxylé / non dégradé correspondant, par exemple l'acide xylarique à partir du D-xylose, et,
- non majoritairement, un produit tricarboxylé mais également non dégradé; i.e. contenant donc le même nombre d'atomes de carbone que le monosaccharide de départ.

### EXEMPLE 4

Dans le cadre de ces essais, on a opéré selon le descriptif général de l'EXEMPLE 1 si ce n'est que, dans le cas présent, l'on a substitué le D-glucose par différentes compositions monosaccharidiques.

Le tableau ci-dessous donne, en fonction du ou des composant(s) constituant la matière sèche de la composition monosaccharidique de départ, le ou les type(s) d'isomère de l'acide-2-carboxy-2,3,4-trihydroxypentane-dioïque obtenu (s) dans le milieu réactionnel après électrooxydation, étant entendu que :
- « 1 » signifie l'isomère (1) tel que décrit précédemment,
- « 2 » signifie l'isomère (2) tel que décrit précédemment,
- « 3 » signifie l'isomère (3) tel que décrit précédemment,
- « 4 » signifie l'isomère (4) tel que décrit précédemment,
- à titre d'exemple, « 1 + 2 » signifie un mélange de
« l'isomère (1) » et de « l'isomère (2) » tels que décrits précédemment,
- « NA » signifie « sodium »,
- « K » signifie « potassium »

| MONOSACCHARIDE(S) DE DEPART | ISOMERE(S) OBTENU(S) |
|---|---|
| D-GLUCOSE | 1* + 2 |
| L-GLUCOSE | 3* + 4 |
| D-GALACTOSE | 1 + 3 |
| D-MANNOSE | 2 |
| D-GULOSE | 3* + 4 |
| D-FRUCTOSE | 2 |
| D-SORBITOL | 1* + 2 |
| D-MANNITOL | 2 |
| GLUCONATE DE NA | 1* + 2 |
| GLUCARATE DE K | 1* + 2 |
| 5-CETOGLUCONATE DE NA | 1 |
| 2-CETOGLUCONATE DE NA | 2 |
| MELANGE D-GLUCOSE / GLUCONATE DE NA | 1* + 2 |

| | |
|---|---|
| * isomère majoritaire du mélange d'isomères | |

Ce tableau montre que le procédé conforme à l'invention permet, en jouant sur la nature de la composition monosaccharidique de départ, d'obtenir des compositions polycarboxyliques dont on peut influencer la teneur particulière en chacun des 4 isomères susdécrits de l'acide 2-carboxy-2,3,4-trihydroxypentanedioïque.

Il est à noter que dans le cas d'une composition monosaccharidique à base respectivement de D ou L-galactose, on obtient, en mettant en oeuvre le procédé selon l'invention, des compositions polycarboxyliques dans lesquelles l'isomère obtenu (respectivement isomère (1) ou isomère (3) - cf. tableau ci-dessus) peut être facilement séparé du composé dicarboxylique obtenu conjointement, en l'occurrence le sel dipotassique de l'acide galactarique (ou acide mucique) du fait de la très faible solubilité dans l'eau de ce dernier.

## Revendications

1. Procédé de préparation d'une composition polycarboxylique, **caractérisé par le fait qu'**il comprend une étape au cours de laquelle on soumet une composition monosaccharidique à un traitement d'oxydation électrochimique mené en absence d'hypochlorite de sodium et en présence a) d'un oxyde d'amine et b) d'une anode à base d'un matériau en carbone.

2. Procédé selon la revendication 1, **caractérisé en ce que** ladite anode est à base d'un matériau en carbone présentant une surface spécifique au moins égale à 0,10 m²/g, de préférence au moins égale à 0,20 m²/g.

3. Procédé selon la revendication 2, **caractérisé en ce que** ledit matériau en carbone présente uno surface spécifique au moins égale a 0,25 m²/g.

4. Procédé selon l'une des revendication 2 ou 3, **caractérisé en ce que** ladite anode est choisie dans le groupe comprenant les feutres de carbone et les charbons actifs granulaires.

5. Procédé selon l'une des revendications 1 à 4, **caractérisé en ce que** ledit traitement d'oxydation électrochimique est mené à un pH compris entre 10 et 14, de préférence compris entre 11,5 et 14..

6. Procédé selon la revendication 5, **caractérisé en ce que** le pH est compris entre 12 et 13,5.

7. Procédé selon l'une quelconque des revendications 1 à 6, **caractérisé en ce que** ledit traitement d'oxydation électrochimique est également mené en absence de bromure de sodium.

8. Composition polycarboxylique susceptible d'être obtenue selon l'un quelconque des revendications 1 à 7, **caractérisée en ce qu'**elle contient :
- de 30 à 90 % d'un ou plusieurs produits choisis parmi les acides dicarboxyliques et leurs sels, et
- de 3 à 50 % d'un ou plusieurs produits choisis parmi les acides tricarboxyliques et leurs sels,
ces pourcentages étant exprimés en poids sec par rapport au poids sec total, de ladite composition.

9. Composition polycarboxylique selon la revendication 8, **caractérisée en ce qu'**elle contient :
- de 30 à 90 % d'acide glucarique, sous forme d'acide libre et/ou de sel (s), et,
- de 3 à 50 % d'acide 2-carboxy-2,3,4-trihydroxypentanedioïque, sous forme d'acide libre et/ou de sel(s).

10. Composition polycarboxylique selon la revendication 9, **caractérisé en ce qu'**elle contient au total au moins 90 % d'acide glucarique et d'acide 2-carboxy-2, 3, 4-trihydroxypentanedioïque, ce pourcentage étant exprimé en poids sec total desdits produits par rapport au poids sec total de ladite composition.

11. Acide 2-carboxy-2,3,4-trihydroxypentane-dioïque, ses sels et dérivés.

12. Utilisation d'une composition selon l'une quelconque des revendication 8 à 11
dans les industries des détergents et agents de nettoyage, du traitement des eaux, du traitement des métaux, du traitement des végétaux, du traitement des fibres, en particulier textiles ou papetières, des liants hydrauliques, des adhésifs, de la fonderie, des peintures ou du cuirs, des industriels alimentaires, pharmaceutiques ou cosmétologiques.

## Claims

1. A method for preparing a polycarboxylic composition, **characterized in that** it comprises a step in which a monosaccharide composition undergoes an electrochemical oxidation treatment carried out in the absence of sodium hypochlorite and in the presence of a) an amine oxide and b) a carbon-based anode.

2. The method as claimed in claim 1, **characterized in that** said anode is based on a carbon material having a surface area at least equal to 0.10 m²/g, preferably at least equal to 0.20 m²/g.

3. The method as claimed in claim 2, **characterized in that** said carbon material has a surface area at least equal to 0.25 m²/g.

4. The method as claimed in either of claims 2 or 3, **characterized in that** said anode is selected from the group comprising carbon felts and granular active charcoals.

5. The method as claimed in any one of claims 1 to 4, **characterized in that** said electrochemical oxidation treatment is carried out at a pH of between 10 and 14, preferably of between 11.5 and 14.

6. The method as claimed in claim 5, **characterized in that** the pH is comprised between 12 and 13.5.

7. The method as claimed in any one of claims 1 to 6, **characterized in that** said electrochemical oxidation treatment is also carried out in the absence of sodium bromide.

8. A polycarboxylic composition obtainable by the method as claimed in any one of claims 1 to 7, **characterized in that** it comprises:
- from 30 to 90 % of one or more products selected from dicarboxylic acids and their salts, and
- from 3 to 50 % of one or more products selected from tricarboxylic acids and their salts, these percentages being expressed as dry weight with respect to the total dry weight of said composition.

9. The polycarboxylic composition as claimed in claim 8. **characterized in that** it comprises:
- from 30 to 90 % of glucaric acid, in the free acid form and/or in the form of a salt, and
- from 3 to 50% of 2-carboxy-2,3,4-trihydroxy-pentanedioic acid, in the free acid form and/or in the form of a salt.

10. The polycarboxylic composition as claimed in claim 9, **characterized in that** it comprises in total at least 90% of glucaric acid and of 2-carboxy-2,3,4-trihydroxypentanedioic acid, this percentage being expressed as total dry weight of said products with respect to the total dry weight of said composition

11. 2-Carboxy-2,3,4-trihydroxypentanedioic acid, its salts and derivatives.

12. The use of a composition as claimed in any one of claims 8 to 11 in the following industries: detergents and cleaning agents, water treatment, metal treatment, plant treatment, fibres treatment, in particular textile fibres or paper fibres, hydraulic binders, adhesives, founding, paints or leather, or in the food, pharmaceutical or cosmetic industries.

## Patentansprüche

1. Verfahren zur Herstellung einer Polycarboxyzusammensetzung, **dadurch gekennzeichnet, dass** es eine Stufe umfasst, während welcher eine Monosaccharidzusammensetzung einer elektrochemischen OxidationsBehandlung unterzogen wird, die in Abwesenheit von Natriumhypochlorit und in der Gegenwart von a) einem Aminoxid und b) einer Anode aus einem Kohlenstoffmaterial durchgeführt wird.

2. Verfahren nach Anspruch 1, **dadurch gekennzeichnet, dass** die Anode aus einem Kohlenstoffmaterial besteht, das eine spezifische Oberfläche von mindestens 0,10 m²/g, vorzugsweise mindestens 0,20 m²/g aufweist.

3. Verfahren nach Anspruch 2, **dadurch gekennzeichnet, dass** das Kohlenstoffmaterial eine spezifische Oberfläche von mindestens 0,25 m²/g aufweist.

4. Verfahren nach einem der Ansprüche 2 oder 3, **dadurch gekennzeichnet, dass** die Anode ausgewählt wird aus der Gruppe, umfassend Kohlenstoffvliese und Aktivkohlegranalien.

5. Verfahren nach einem der Ansprüche 1 bis 4, **dadurch gekennzeichnet, dass** die elektrochemische Oxidationsbehandlung bei einem pH-Wert von zwischen 10 und 14, vorzugsweise zwischen 11,5 und 14, durchgeführt wird.

6. Verfahren nach Anspruch 5, **dadurch gekennzeichnet, dass** der pH-Wert zwischen 12 und 13,5 liegt.

7. Verfahren nach einem der Ansprüche 1 bis 6, **dadurch gekennzeichnet, dass** die elektrochemische Oxidationsbehandlung gleichfalls in der Abwesenheit von Natriumbromid erfolgt.

8. Polycarboxyzusammensetzung, die nach einem der Ansprüche 1 bis 7 erhalten werden kann, **dadurch gekennzeichnet, dass** sie enthält:
- von 30 bis 90 % eines oder mehrerer Produkte, ausgewählt aus den Dicarbonsäuren und ihren Salzen, und
- von 3 bis 50 % eines oder mehrerer Produkte, ausgewählt aus den Tricarbonsäuren und ihren Salzen, wobei diese Prozentgehalte in Trockengewicht bezogen auf das Gesamttrockengewicht der Zusammensetzung angegeben sind.

9. Polycarboxyzusammensetzung nach Anspruch 8, **dadurch gekennzeichnet, dass** sie enthält:
- von 30 bis 90 % Glucarsäure in der Form der freien Säure und/oder von Salz (Salzen) und
- von 3 bis 50 % 2-Carboxy-2,3,4-trihydroxypentandisäure in der Form der freien Säure und/oder von Salz (Salzen).

10. Polycarboxyzusammensetzung nach Anspruch 9, **dadurch gekennzeichnet, dass** sie insgesamt mindestens 90 % Glucarsäure und 2-Carboxy-2,3,4-trihydroxypentandisäure enthält, wobei dieser Prozentgehalt in Trockengewichten der Produkte bezogen auf das Gesamttrockengewicht der Zusammensetzung angegeben ist.

11. 2-Carboxy-2,3,4-trihydroxypentandisäure, ihre Salze und Derivate.

12. Verwendung einer Zusammensetzung nach einem der Ansprüche 8 bis 11 in der Industrie der Wasch- und Reinigungsmittel, der Wasserbehandlung, der Metallbehandlung, der Pflanzenbehandlung, der Faserbehandlung, insbesondere Textilien oder Papier, der hydraulischen Bindemittel, der Klebstoffe, der Gießerei, der Anstrichmittel oder Leder, der Lebensmittelindustrie, pharmazeutischen oder kosmetischen Industrie.
